# EUROPEAN PATENT SPECIFICATION

(11) **EP 4 112 737 B1**
(45) Date of publication and mention of the grant of the patent: **20.03.2024**
(21) Application number: 22182918.7
(22) Date of filing: 04.07.2022
(51) Int. Cl.: C12Q 1/60, G01N 33/52, G01N 33/92

(54) **DEVICE FOR TESTING BLOOD PLASMA**
VORRICHTUNG ZUM TESTEN VON BLUTPLASMA
DISPOSITIF DE TEST DE PLASMA SANGUIN

(30) Priority: 02.07.2021 EP 21275092
(43) Date of publication of application: 04.01.2023
(73) Proprietor: Vital Signs Solutions Limited, Cambridge Cambridgeshire CB4 0FW (GB)
(72) Inventor: Kalikavunkal, Prameen, Cambridge CB4 0FW (GB)
(74) Representative: Williams Powell

(56) References cited:
- EP-A1- 2 040 073
- WO-A1-03/056163
- WO-A1-2008/086019
- WO-A1-2017/161350
- WO-A1-2018/025041

## Description

### Field of the invention

The present invention relates to a device for testing blood plasma, and in particular to a device for testing blood plasma for cholesterol.

### Background of the invention

Cardiovascular diseases are a major cause of death worldwide. Total cholesterol (TC), triglycerides (TGL), and high density cholesterol (HDL) (sometimes referred to as "good cholesterol") are important biomarkers to ascertain the risk of suffering from cardiovascular conditions in the future. Testing regimes for the amounts of these different types of cholesterol - and the ratio between them - are commonly used to predict a patient's future health profile.

Common cholesterol test devices measure one of the three kinds of cholesterol from a single drop of blood (10µl). Some test kits integrate all the tests into one and produce the individual results from a few drops of blood (50µl). These tests kits require additional expensive equipment for the measurement and analysis.

WO 03,056,163A1 discloses a test strip and method for determining HDL concentration from whole blood or plasma. WO 2008/086,019A1 discloses a device and method for measuring LDL-associated cholesterol.

EP 2,040,073A1 discloses a microfluidic device and method for fluid clotting time determination. It is disclosed that, in a preferred embodiment, this microfluidic device includes capillary channels (6a, 6b) which are in a curved shape, most preferably having a serpentine shaped track, in order to minimize the area of the device while maintaining the length of the channels.

WO 2017/161,350A1 discloses a microfluidic device, system, and method.

WO 2018/025,041A1 discloses a device and method for liquid analysis to detect biomarkers.

The present application seeks to provide an improved cholesterol testing device.

### Summary of the invention

The invention is defined in the appended claims.

In accordance with a first aspect of the present invention, there is provided a device for testing blood plasma for cholesterol, including an inlet for blood plasma, at least one test region which includes a test for identifying high density lipoprotein (HDL) cholesterol, first and second opposing walls defining at least one channel, wherein said channel has a first end proximate the inlet and a second end proximate the test region, a transfer element which is formed of a material which allows capillary flow of blood plasma from the inlet along said channel to the test region, a reactant located in said channel which reacts with low density lipoprotein (LDL) cholesterol in said blood plasma, and prevents the LDL cholesterol from reaching the test region, wherein said channel has a multiplicity of corners on one or both of said walls, said corners defining a zigzag profile which affects the flow of blood plasma in order to promote the reaction of the plasma with said reactant.

The corners may define an irregular zigzag profile.

In some embodiments, the corners define an interior angle from 70-90° and an exterior angle from 90-140°.

The width of the channel may be non-uniform along its length.

Preferably, the channel includes a density of corners of at least two corners for each mm of the length of the channel.

The total number of corners is preferably at least 14, more preferably from 16 to 32, and most preferably 16.

In preferred embodiments, the average width of the channel is from 18% to 21% of the length of the channel, the maximum width of the channel does not exceed 1.9 times the average width, the minimum width of the channel is not less than 48% of the average width, and the depth of the channel is about 7% of the average width.

The device may additionally include a test region which includes a test for identifying total cholesterol and additionally include a second channel, wherein said channel has a first end proximate the inlet and a second end proximate said test region, and wherein the transfer element allows capillary flow of blood plasma from the inlet along said channel to said test region.

The device may additionally include a test region which includes a test for identifying triglycerides and additionally include a third channel, wherein said channel has a first end proximate the inlet and a second end proximate said test region, and wherein the transfer element allows capillary flow of blood plasma from the inlet along said channel to said test region.

The device may additionally include a region of said transfer element between the inlet and the end of each channel proximate the inlet.

The transfer element preferably has hydrophobic and hydrophilic regions which are patterned so as to define the at least one channel and the at least one test region. Preferably, each channel and test region is formed from the hydrophilic regions.

The transfer element is preferably formed from a porous membrane.

In a preferred embodiment, the device additionally includes a filter for separating blood plasma from whole blood, the filter being located on the opposite side of the inlet to the at least one channel. The device may have a single transfer element. It is preferred that the transfer element is substantially planar, wherein the capillary flow of blood plasma is in the plane of the transfer element.

In accordance with a second aspect not part of the present invention, there is provided a device for testing blood plasma for cholesterol, including an inlet for blood plasma, a separation element in fluid communication with the inlet, wherein the separation element is formed of a material which allows capillary flow of blood plasma, wherein the separation element includes a first channel for capillary flow of blood plasma from the inlet to a first test region, a second channel for capillary flow of blood plasma from the inlet to a second test region, and a third channel for capillary flow of blood plasma from the inlet to a third test region, wherein the third channel includes a reactant for reacting with low density lipoprotein (LDL) cholesterol, thereby preventing it from reaching the third test region, and wherein the first test region includes a test for identifying total cholesterol, the second test region includes a test for identifying triglycerides and the third test region includes a test for identifying high density lipoprotein (HDL) cholesterol.

A technical advantage of the preferred embodiment of the present invention is the provision of a single device which is able to determine the concentration of total cholesterol, HDL and Triglyceride concentrations from a single prick of whole blood (about 15µL or 20 µL) without the use of any anticoagulants.

The device is engineered to extract plasma from whole blood using a 3 layered lateral flow assay model.

Preferably, the transfer element is formed from a cellulose membrane. Preferably this is substantially planar.

The transfer element (which is preferably formed from a nitrocellulose membrane) may have hydrophobic and hydrophilic regions which are configured so as to define said channels and test regions, with the hydrophilic regions forming said channels and test regions.

The hydrophobic regions may be formed by including a wax on the transfer element. Other methods of forming the hydrophobic regions include spray painting hydrophobic molecules (such as silanes) onto the transfer element or printing melted paraffin wax using a custom-built printer.

In a preferred embodiment, the device additionally includes a filter for separating blood plasma from whole blood, the filter being located on the opposite side of the inlet to said channels. The filter preferably includes at least two filtration layers. The first filtration layer may be a polymer mesh (preferably a nylon mesh) and the second layer may be a glass fibre mesh (preferably an LF1 membrane). In a particularly preferred embodiment, the filter has a tapered part in communication with said inlet.

The individual pores in the nylon mesh acts as a capillary matrix to transfer the fresh whole blood onto a tapering LF1 membrane below it. The LF1 membrane separates the whole blood into plasma and blood cells, and the tapering end conveys the plasma separated onto the nitrocellulose membrane. The tapering section allows the plasma to accumulate and flush into the nitrocellulose membrane below it.

In accordance with a third aspect not part of the invention, there is provided a device for testing blood plasma for cholesterol, including an inlet for blood plasma, a flow element in fluid communication with the inlet, wherein the flow element comprises one or more channels and is formed of a material which allows capillary flow of blood plasma, a testing site for testing the blood plasma in fluid communication with the flow element, and a filter for separating blood plasma from whole blood, the filter being located on the opposite side of the inlet to said channels, the filter including at least two filtration layers, wherein the filter has a tapered part in communication with said inlet.

Preferably the filtration layers are as defined in the second aspect of the invention.

According to a fourth aspect, there is provided a method of testing blood plasma for cholesterol, including: depositing a sample of blood plasma onto the device of any preceding aspect, wherein the sample is flowed by capillary forces from the inlet to the at least one test region of said device through the at least one channel; and, determining a concentration of cholesterol present in the sample according to a colourimetric analysis of the at least one test region.

The method may include depositing onto said device a quantity of whole blood which includes said sample of blood plasma, the filter of said device separating blood plasma from said whole blood. Said quantity of whole blood may be a single droplet of blood from a fingerprick. Said quantity of whole blood may be from 15 to 25 µL, preferably about 20 µL.

The method may include determining the concentration of any of triglycerides, total cholesterol, and HDL cholesterol or any combination thereof present in said blood plasma sample by colourimetric analysis of respective test regions of said device.

The method may include conducting said colourimetric analysis 6 minutes after deposition of said blood sample onto said device.

### Specific description

A number of preferred embodiments of the invention will now be described, with reference to and as illustrated in the accompanying drawings, in which:
Figure 1 is an exploded view of an embodiment of the device;
Figure 2 is a plan view of an embodiment of a transfer element (200) suitable for use with the device of Figure 1;
Figure 3 is a photograph of a portion of a channel (230) of the transfer element of Figure 2;
Figure 4 is an illustration of different configurations of channels within a transfer element (200);
Figure 5 is an illustration of the capillary flow regimes through the different channel designs shown in Figure 4;
Figure 6 is a chart which shows the degree of precipitation of LDL cholesterol achieved by the device of Figure 1 when used with transfer elements embodying each of the different channel designs of Figure 4;
Figure 7 is an illustration of various geometries of a blood separation filter (100) suitable for use with the device of Figure 1;
Figure 8 is a collection of graphs showing the performance of the different blood separation filter designs of Figure 7 when incorporated in the device of Figure 1.

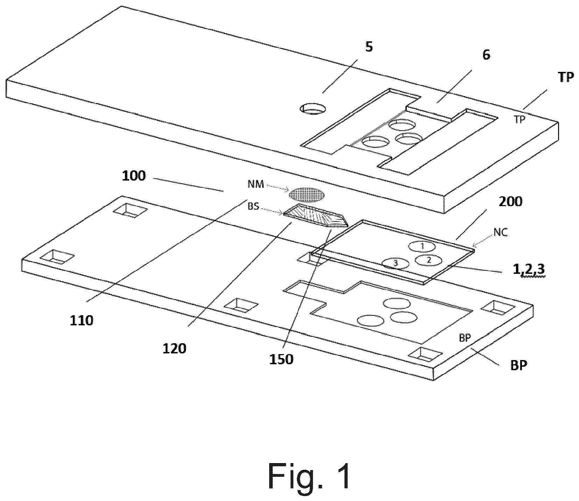

The device as shown in Figure 1 has a top piece (TP) and a bottom piece (BP), each of which is substantially planar. The top and bottom pieces (TP, BP) are configured to interlock with one another such that a unitary body or housing is formed when they are locked together.

Sandwiched between the top and bottom pieces (TP, BP) are a filter (100) and a transfer element (200), which are received within a recess of the bottom piece (BP). Disposed in the top piece (TP) is a blood inlet port (5), through which a user can deposit a sample of whole blood (i.e. red blood cells, white blood cells, platelets, and blood plasma) into the device for assay. Once deposited into the inlet port (5), blood plasma from the sample travels through the filter (100) and the transfer element (200) by capillary action to reach each of three test regions (1, 2, 3). The first test region (1) includes a test for identifying total cholesterol, the second test region (2) includes a test for identifying triglycerides, and the third test region (3) includes a test for identifying high density lipoprotein (HDL) cholesterol. The top piece (TP) also includes a plurality of additional ports (6) which are sized and positioned to give access to each of the three test regions (1, 2, 3).

The filter (100) is disposed between, and in fluid communication with each of, the blood inlet port (5) and the transfer element (200). The function of the filter (100) is to separate the blood plasma from a volume of whole blood deposited into the blood inlet port (5).

The filter (100) comprises first and second filtration layers (110, 120). The first filtration layer (110) is located adjacent to the blood inlet port (5) and is formed of a polymer mesh, such as a nylon mesh. Pores in the polymer mesh of the first filtration layer (110) act as a capillary, enabling whole blood to flush evenly across the filter (100). The second filtration layer (120) is located beneath the first layer (110) and is formed of a glass fibre mesh, such as an LF1 membrane. The second filtration layer (120) separates blood plasma from the whole blood laterally across the glass fibre mesh. An LF1 membrane, in particular, enables a high retention of blood cells compared with other fibre meshes.

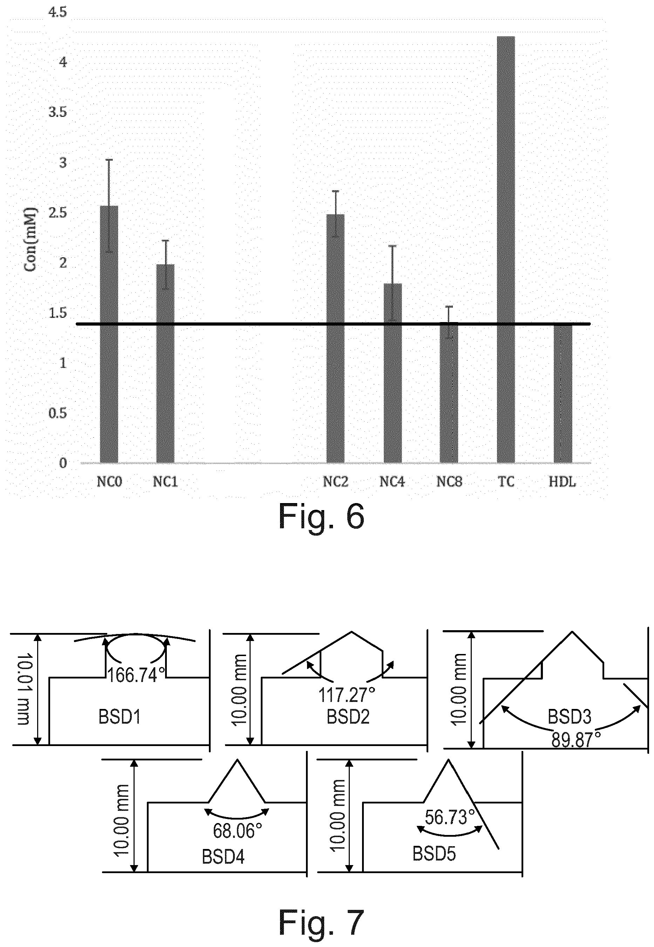

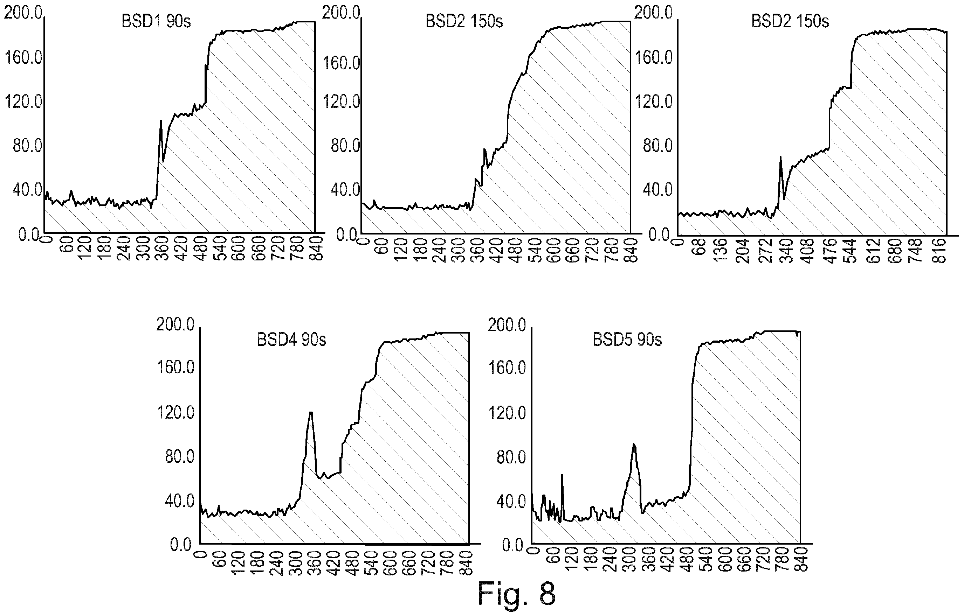

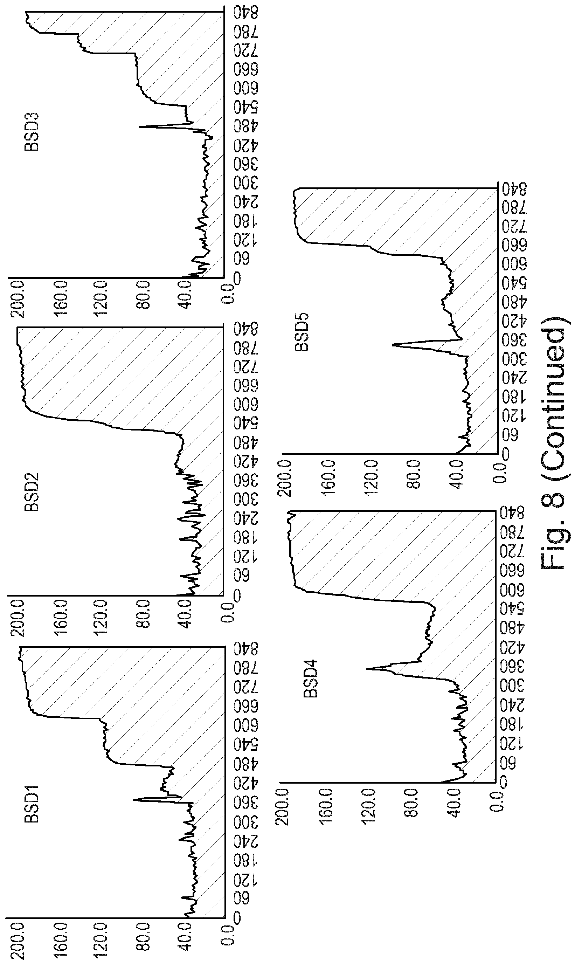

The second filtration layer (120) has a first broad end and a second end which narrows to form a tapered part (150). The tapered part (150) is in communication with an inlet (10) at the transfer element (200). The broad end of the second filtration layer (120) is shaped and sized to allow all the blood from the first filtration layer (110) to be transferred to the second filtration layer (120). The tapered part (150) at the second end is shaped to constrict the blood cells in the glass fibre mesh and concentrate blood plasma at the tip of the taper, where it is then transferred onto the transfer element (200) via the inlet (10). As is described in more detail below with reference to Figures 7 and 8, various angles of taper can be employed.

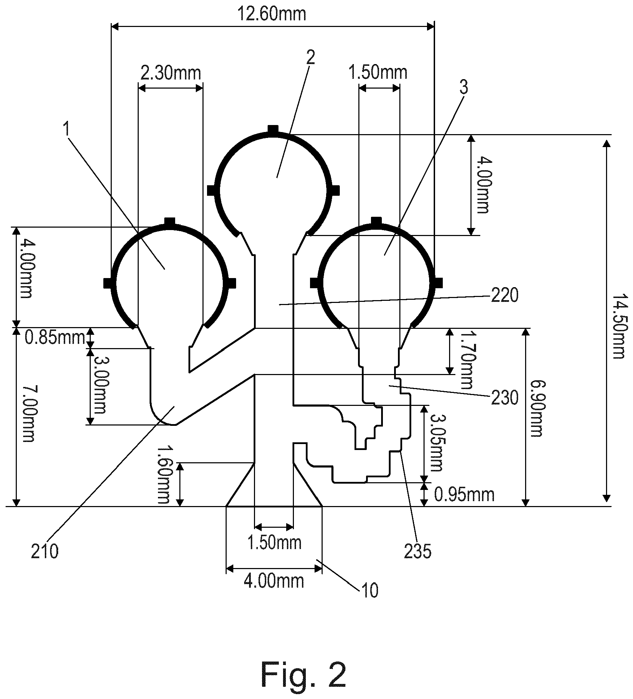

Referring now to Figure 2, an embodiment of the transfer element (200) will be described. The transfer element (200) is substantially planar and formed of a material which allows capillary flow of blood plasma, such as a nitrocellulose membrane.

As described above, an inlet (10) enables a flow of blood plasma into the transfer element (200) from the filter (100). The transfer element (200) comprises first, second, and third elongate channels (210, 220, 230) configured to effect capillary flow of blood plasma from the inlet (10) to each of the first, second, and third test regions (1, 2, 3). The transfer element (200) has hydrophobic and hydrophilic regions which define the channels and test regions (1, 2, 3), with the hydrophilic regions forming said channels and test regions (1, 2, 3).

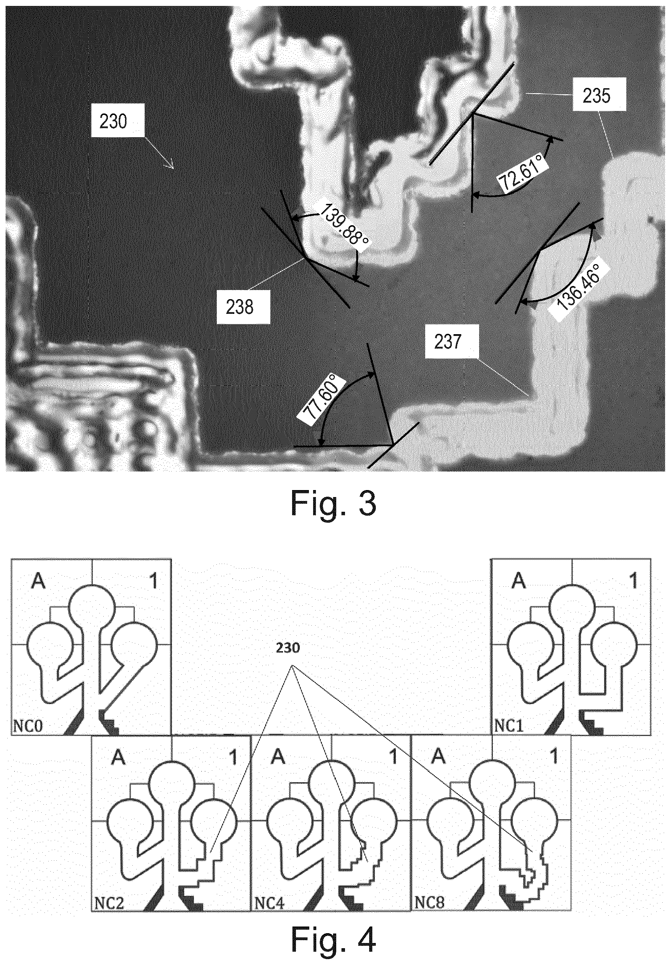

The arrangement of hydrophobic and hydrophilic regions can be formed by additive methods or removal methods. For example, hydrophobic regions can be formed additively by applying a hydrophobic material, such as a wax or hydrophobic ink, which may for example be melted or sprayed onto the nitrocellulose membrane. Alternatively, removal methods include, for example, selective laser ablation of nitrocellulose material from the surface of the membrane. In the embodiment shown in Figure 3, the channels have been formed by selective laser ablation.

Each of the channels (210, 220, 230) is defined by first and second opposing walls, each channel having a first end proximate the inlet (10) and a second end proximate the respective test region (1, 2, 3).

Each of the channels (210, 220, 230) has a different degree of tortuousness, thereby differently affecting the flow of blood plasma therethrough. The second channel (220) is substantially straight. The path of the first channel (210) includes a single corner (that is, the opposing walls of the first channel together comprise a matching pair of corners). The third channel (230) is more tortuous than each of the first and second channels (210, 220), comprising an abundance or multiplicity of corners (235).

The third channel (230) is also longer than each of the first and second channels (210, 220) in order to increase the time taken for blood plasma to reach the third test region (3). Figure 2 illustrates the dimensions of a typical embodiment of the transfer element (200). For the embodiment illustrated in Figure 2, a volume of 3.5µL of blood plasma is required to wick through the entire hydrophilic region of the transfer element (200).

The tortuousness of the third channel (230) also slows down capillary flow of the blood plasma to the third test region (3) by comparison with the flow through the first and second channels. More importantly, however, as is described in detail below, the corners (235) of the third channel (230) also increase mixing of the capillary flow of blood plasma so as to promote the reaction of the plasma with a reactant located in the channel.

The plasma sample contains low density lipoproteins (LDL) and high density lipoproteins (HDL). To determine accurately the amount of HDL in the sample, the LDL from the plasma has to be prevented from reaching the HDL testing region. A reactant (i.e. one or more precipitation agents for example dextran sulphate, phosphotungistic acid, magnesium sulphate, magnesium chloride, or a combination thereof) is therefore located in the third channel (230) and reacts with low density lipoprotein (LDL) cholesterol, thereby preventing it from reaching the third test region (3).

The reactant is dispensed in matrix blocks or occupies micro-pores in the nitrocellulose membrane in the channel (230). The presence of the reactant affects the capillary flow through the membrane. Where the pores are occupied, the capillary flow avoids the pores containing precipitation agents by meandering around them or having a delayed flow into the regions containing the precipitation agent. This can lead to incomplete and irregular precipitation of LDL from the plasma, prejudicing the accuracy of the test.

Flow inside the nitrocellulose membrane can be directional and occurs due to capillary forces. The flow profile is therefore also somewhat parabolic, with the liquid wicking faster along the middle of the hydrophilic region than along the edges (near the walls). This can also lead to incomplete and irregular precipitation of LDL from the plasma, prejudicing the accuracy of the test.

The corners (235) are distributed along the walls of the third channel (230), defining a zigzag profile. The zigzag profile redirects capillary flow of blood plasma at the walls of the channel toward the middle of the width of the channel. In this way, the capillary flow of plasma is "kneaded" inward in the channel such that slower flow near the walls is brought into the middle of the channel, increasing exposure of the LDL cholesterol in the plasma sample to the reactant. That is, the corners (235) promote mixing between the slow moving liquid along the edges and the faster moving liquid in the middle of the hydrophilic regions. The corners thereby also guide the plasma to flow into the areas of dispensed reactant in the channel, promoting precipitation of the LDL.

Each corner (235) is an angular vertex in a wall of the channel. Ideally, each of the corners (235) is right-angled. However, due to manufacturing tolerances at the microscale of the corners, this is not always achievable with precision. In practice, inward corners may have angles from 70-90 degrees and outward corners may have angles from 90-140 degrees. For example, in the embodiments shown, it has been observed that inward corners (237) of the zigzag have angles from 70-80 degrees and outward corners (238) of the zigzag have angles from 130-140 degrees, measured at the interior of the channel. Figure 3 shows, by way of example, observed dimensions of two inward corners (237) and two outward corners (238) in an embodiment of the transfer element (200). In this example, two of the inward corners have angles of 77.60 and 72.61 degrees, respectively, and two of the outward corners have angles of 139.88 and 136.46 degrees, respectively. As is described above, the corners (235) define a zigzag profile which promotes the reaction of plasma with the reactant in the channel. The density of corners present in the channel significantly affects the level of mixing in the channel and therefore the efficiency of precipitation. The density of corners means the number of corners per unit length of the channel for a given width and depth of the channel.

The preferred embodiment shown in Figure 2 has a density of corners that has been found to enable at least substantially complete precipitation of the LDL cholesterol in the volume of blood plasma that reaches the third test region (3) by travelling along the channel.

Complete precipitation means precipitation of all the LDL cholesterol from the volume of blood plasma that reaches the test region. Substantially complete precipitation means at least near enough to complete precipitation of the LDL cholesterol in the volume of blood that any remainder, if present, will not (significantly) prejudice the accuracy or outcome of the test at the test region. The National Cholesterol Education Program (NCEP) provides that a clinically acceptable margin of error in HDL measurement is 12%. Therefore, substantially complete precipitation may be removal of 88% or more of the LDL cholesterol in the volume of plasma that reaches third test region.

In the preferred embodiment, the multiplicity of corners includes a total of 16 corners, eight corners being disposed in each wall, the third channel having a length of 7-8mm. The channel in this embodiment therefore has a density of corners of about two corners for each mm of the length of the channel, the channel having a depth of 100 µm, an average width of 1.45mm, a minimum width of 0.75mm, and a maximum width of 2.73mm. In this embodiment, the volume of plasma that fills the third channel is 1-2µL, 1-1.2µL of which is required to fill up the test region.

The length of the channel is measured along the middle of the channel, following the path of the channel, from the first end to the second end. The width of the channel means the distance between opposing first and second walls of the channel, measured in an axis transverse to the path of the channel. The width of the third channel is preferably not uniform along its length. In particular, corners of the multiplicity variously constrict and expand the width of the third channel. Therefore, the average width, the minimum width, and the maximum width of the channel are relevant. The depth of the channel means the depth of the hydrophilic region in the channel, through which capillary flow travels.

The improved efficiency of precipitation obtained at this number of corners per unit length is preserved when scaled (to channels of varying lengths), provided the relative cross-sectional dimensions of the channel are maintained (and, of course, that the dimensions are also practicable for performing the test). Therefore, the density of corners can be given as a number of corners for each mm of the length of the channel for a given ratio of cross-sectional dimensions of the channel. For example, the density of corners of the preferred embodiment is 2 corners per mm of length of the channel, wherein the average width of the channel is from 18% to 21% of the length of the channel, the maximum width of the channel does not exceed 1.9 times the average width, the minimum width of the channel is not less than 48% of the average width, and the depth of the channel is about 7% of the average width.

Further dimensions of the preferred embodiment of the transfer element (200) are provided at Figure 2.

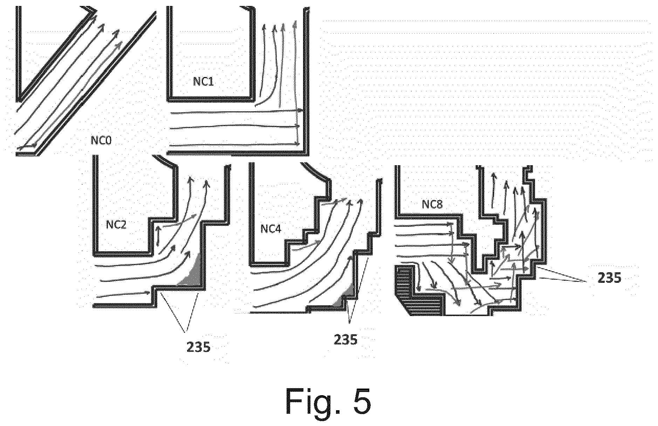

Figure 4 illustrates a series of transfer elements having various numbers of corners in the third channel. The different designs are denoted by the number of corners present in each wall of the third channel: NC0 has a straight channel with no corners; NC1 has a channel with a single corner in each wall; NC2 has a channel with a pair of corners in each wall; NC4 has four corners in each wall; and, NC8 has eight corners in each wall. Figure 5 illustrates the flow regimes of capillary flow of blood plasma in each of these channels. In general, mixing of the capillary flow is increased with increasing number of corners in the channel, according to the mechanism described above.

HDL tests were performed using the device of Figure 1 in combination, in turn, with each of the transfer elements of Figure 4. A chart of the HDL readings obtained at the test region is provided at Figure 6, which shows a comparison of the HDL measured at each test region with that known to be present in the sample. The chart indicates (as a vertical red bar) the amount of cholesterol (both LDL and HDL) detected at the test region for each variant, and (as a horizontal golden bar) the true amount of HDL in the sample. A further vertical bar TC indicates the total cholesterol (HDL and LDL) present in the deposited samples. The discrepancy between the red and golden bars for each variant indicates the proportion of LDL that was not precipitated in the channel.

Having no corners in the channel leading to the HDL test region does precipitate a certain amount of LDL but not all. Increasing the number of corners increases the precipitation efficiency (up to a limit). The preferred embodiment of the device presented here uses 16 corners to achieve the desired precipitation. Channels containing 12 or fewer corners demonstrated incomplete precipitation of the LDL. Further variants denoted NC5 (having 10 corners, five of which on each wall) and NC6 (having 12 corners, six of which on each wall) showed precipitation but the measured values were 40% and 20% higher than the actual HDL values. This indicates that not all the LDL from the blood sample was precipitated. There was no particular trend noticed in the intervening cases between NC0 (having no corners) to NC6. A variant designated NC7 (having 14 corners, seven of which on each wall) provided HDL values 13-15% higher than the actual value, indicating incomplete precipitation of LDL from the sample but close to the clinically acceptable margin of error. As described above, NC8 (having a zigzag with a total of 16 corners, eight of which are disposed on each wall) has been found to provide complete precipitation of the LDL in the plasma at the test region.

At higher numbers of corners still, greater volumes of blood plasma become necessary to complete the test. A transfer element (designated NC16) with 32 corners in the channel, 16 of which are disposed on each wall, failed to produce results for the given volume and the conditions of the tests.

Intuitively, the distribution of corners along the channel is also influential. That is, if corners are grouped only at the start of the channel or only toward the end of the channel, then the utility of the corners is lessened (as there is less opportunity for flow redirected at the corners to interact with the reactant). In channel variants where the corners were grouped in this way (rather than being distributed along substantially the length of the channel), the accuracy of the HDL test at the test region was found to be compromised. The latter case (where the corners are grouped only at the entrance to the test region) in fact showed little to no precipitation of LDL. This appears to happen due to the fast moving liquid in the middle reaching the test sites delivering the LDL and HDL molecules for the enzymatic reactions in the test sites. The multiplicity of corners is therefore preferably distributed along the channel, more preferably along substantially the whole length of the channel.

It will be appreciated, however, that the multiplicity of corners (235) is not necessarily distributed evenly on both walls of the channel. In the preferred embodiment shown in Figure 2, for example, the third channel (230) provides an arc from its first end to its second end. The corners on the wall at the interior of the curve are therefore more closely grouped than are the corners on the opposing wall. Some of the corners (235) are mirrored or duplicated by a corresponding corner on the opposing wall of the channel. Some of the corners are staggered between the opposing walls of the channel.

In the embodiments shown, the multiplicity of corners (235) includes corners in both of first and second opposing walls of the third channel (230). With reference to Figure 2, for example, the first and second walls in this preferred embodiment have an equal number of corners, each having eight corners. However, in other embodiments, there can be more corners on one wall than on the other wall. Optionally, there can be corners along only one wall of the channel.

A channel with corners on only one wall, the opposing wall being smooth, has been found to achieve some precipitation, but a lesser amount than is achieved by a channel with the same number of corners distributed between both walls.

The influence of a number of further modifications (relative to the preferred embodiment of Figure 2) has been investigated. When the channel is halved in width and doubled in length the device does not produce any results for HDL. This is probably due to a combination of thin channels, and the precipitated lipoprotein blocking the flow of liquid. Dispensing of chemicals in such a small arm width also becomes problematic. When the channel is doubled in width and halved in length, it becomes difficult for the capillary flow to reach the test region for the same volume of blood plasma. Reducing the length of the HDL channel while increasing the width cuts off the test sites from communicating with the inlet of the device. On further increasing the length of the channel to connect to the HDL site, the volume of the sample required increases.

With reference now to Figure 7, variants of the blood separation filter (100) are described. The blood separation filter (100) helps in separating the plasma from the whole blood.

### Requirements for a blood separation membrane.

1. The blood cells should be retained in the blood separation membrane.
   The blood separation membrane (BSM) is part of a device that works based on colorimetric reactions. Change in the colour of the test sites gives an indication to the concentrations. If blood cells are not retained in the separation region, they can reach the test sites and give false positives. Red blood cells, if they enter into the nitrocellulose membrane which have branches leading to the test site, could influence the capillary flow of the plasma from the inlet to the test sites.
2. The plasma transfer from the BSM to the nitrocellulose membrane should not be fast.
   The full functioning (precipitation of LDL, enzymatic activity) in the device takes 6mins to finish. If the device has all the test sites filled up too quickly (e.g. <2mins) the reactions becomes incomplete and the test fails.

The tapering end of the filter (100) plays an important role in transferring the plasma onto the transfer element (200). A 1mm x 1mm BSM has the capacity to wick 0.2-0.3µL of the blood/plasma solution. Varying the size/angle or the tapering of the filter (100) controls i) the collection of plasma to the tapered end and transfer of the plasma to the transfer element (200), ii) the amount of time required to fill up the test region(s) via the transfer element (200), iii) the volume required to fill up the test region(s) via the transfer element (200), and iv) the separation efficiency of lipoproteins in the transfer membrane (200).

Figure 7 shows various geometries of tapered end for the blood separation filter. The variants are denoted in order of increasing sharpness of the taper: BSD1 has a comparatively blunted narrow end with an angle of 166.74 degrees; BSD2 has a narrow end with an angle of 117.27 degrees; BSD3 has a narrow end with an angle of 89.87 degrees; BSD4 has a narrow end with an angle of 68.06 degrees; and, BSDS has a comparatively sharp narrow end with an angle of 56.73 degrees.

At Figure 8, graphs are collected which show the performance of the different blood separation filter designs of Figure 7 when incorporated in the device of Figure 1. Low flat lines on the x axis indicate the amount of coverage of the blood cells. The longer the low flat lines on the x-axis the more blood cells have been transferred to the nitrocellulose membrane of the transfer element. The top five graphs indicate the amount of blood cells that have crept into the transfer element at the moment the devices were filled up, and the bottom five graphs indicate the amount of blood cells present at the end of the test duration (6mins).

Higher angles (180-150 degrees) for the tapering end function like having a circular end, which produces good separation since more membrane matrix is available, but fills up the test regions in under 90s. This speed of transfer also prevents blood cells from creeping into the transfer element.

Angles from 120-80 degrees produce good separation of the plasma from the blood cells, and conserve plasma because of the reduction in the matrix volume. However, small amounts of red blood cells are able to creep into the transfer element in this configuration, although the amount is not significant to interfere with the test/functioning of the device. The best combination of effective plasma separation along with a desirable time for the test regions to be filled was achieved with angles close to 90 degrees. Therefore a taper of about 90 degrees is adopted in the preferred embodiment of the filter (100).

Angles below 70 degrees usually fill up the device with plasma after separation in under 90 seconds and also allow a lot more red blood cells to transfer onto the transfer element.

The device is advantageously physico-chemical and requires no electronics to generate a readable output, nor does it generate any energy of note itself. It utilises the ability of liquid (blood or plasma) to move across porous material by capillary forces.

The operation of the device according to the preferred embodiment described above is set out sequentially below:
Step 1. 20µL of whole blood (from a fingerprick) is transferred with a calibrated micropipette from the finger of a human onto the blood inlet port (5).
Step 2. The deposited whole blood is quickly spread over a circular area (Ø 6mm) of the first filtration layer (110), which comprises a high-porosity nylon mesh membrane, and descends by gravity and capillary forces onto the second filtration layer (120), which comprises an LF1 glass fibre membrane. Red blood cells, white blood cells, and platelets are then separated from the whole blood as the blood starts moving laterally across the second filtration layer (120). From the 20 µL of whole blood deposited at the inlet, 7-8 µL of plasma is extracted.
Step 3. The tip of the tapered part (150) of the second filtration layer (120) is in communication with the transfer element (200), which comprises a nitrocellulose membrane, via inlet (10). Blood plasma (i.e. whole blood depleted of blood cells and platelets), continues flowing on the transfer element (200) by capillary forces on the nitrocellulose membrane and the flow is split into three channels (210, 220, 230). 1-1.2 µL of blood plasma reaches and fills each test region. The remainder of the blood plasma remains in the channels.
Step 4. Plasma flowing through the first channel (210) reaches the first test region (1) within 2-3 minutes, and all cholesterol and cholesterol esters molecules present in the plasma react with reagents infused at the test area to produce a distinct purple colour, the intensity of which is proportional to the concentration of triglycerides in the plasma sample.
Step 5. Plasma flowing through the second channel (220) reaches the second test region (2) within 2-3 minutes, and triglycerides present in the plasma sample react with reagents infused at the test area to produce a distinct purple colour, the intensity of which is proportional to the concentration of total cholesterol in the plasma sample.
Step 6. The third channel (230) comprises 16 corners (235), eight of which are disposed on each wall of the channel. The corners (235) define a zigzag profile. Blood plasma flows through the third channel (230) and is directed by the zigzag profile of the channel walls to interact with a reactant (in this case, a combination of phosphotungstic acid and magnesium sulphate) infused in the channel. The reactant reacts with low-density lipoprotein (LDL) cholesterol in the blood plasma, thereby preventing it from reaching the third test region (3). Therefore, only high-density lipoproteins (HDL) continue flowing to the test region (3), enabling the detection of the HDL portion of the blood sample at the third test region (3).
Step 7. Plasma flowing through the third channel (230) reaches the third test region (3), usually within 3-4 minutes, and only HDL present in the plasma sample reacts with the reagents infused at the test area to produce a distinct purple colour, the intensity of which is proportional to the concentration of HDL in the plasma sample.
Step 8. After 6 minutes has elapsed from the deposition of the blood sample - being sufficient time for the optimum purple colour to have developed in each of the test regions (1, 2, 3) - a colourimetric analysis can be conducted.

It will be appreciated that, while the device of Figure 1 comprises a filter (100) and transfer element (200), the device may be used without a filter (100), in which case a sample of blood plasma may be deposited directly onto the transfer element (200). Although preferred embodiments of the filter (100) are described, the device may comprise a transfer element (200) as described above and any suitable blood separation filter. Similarly, embodiments of the device may comprise a filter (100) as described above and any suitable transfer element. It will also be appreciated that alternative embodiments of the transfer element (200) may not include either or both of the first or second test regions (1, 2) and corresponding channels (210, 220).

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

## Claims

1. A device for testing blood plasma for cholesterol, including
an inlet (10) for blood plasma,
at least one test region (3) which includes a test for identifying high density lipoprotein (HDL) cholesterol,
first and second opposing walls defining at least one channel (230), wherein said channel has a first end proximate the inlet and a second end proximate the test region,
a transfer element (200) which is formed of a material which allows capillary flow of blood plasma from the inlet along said channel to the test region,
a reactant located in said channel which reacts with low density lipoprotein (LDL) cholesterol in said blood plasma, and prevents the LDL cholesterol from reaching the test region (3),
wherein said channel has a multiplicity of corners (235) on one or both of said walls, said corners defining a zigzag profile which affects the flow of blood plasma in order to promote the reaction of the plasma with said reactant.

2. A device as claimed in claim 1, wherein the corners (235) define an irregular zigzag profile.

3. A device as claimed in claim 1 or 2, wherein the corners (235) define an interior angle from 70-90° and an exterior angle from 90-140°.

4. A device as claimed in any preceding claim, wherein the width of the channel (230) is non-uniform along its length.

5. A device as claimed in any preceding claim, wherein the channel (230) includes a density of corners (235) of at least two corners for each mm of the length of the channel.

6. A device as claimed in any preceding claim, wherein the total number of corners (235) is at least 14, preferably from 16 to 32, and most preferably 16.

7. A device as claimed in any preceding claim, wherein the average width of the channel (230) is from 18% to 21% of the length of the channel, the maximum width of the channel does not exceed 1.9 times the average width, the minimum width of the channel is not less than 48% of the average width, and the depth of the channel is about 7% of the average width.

8. A device as claimed in any preceding claim, additionally including a test region (1) which includes a test for identifying total cholesterol and additionally including a second channel (210), wherein said channel has a first end proximate the inlet (10) and a second end proximate said test region, and wherein the transfer element (200) allows capillary flow of blood plasma from the inlet along said channel to said test region.

9. A device as claimed in any preceding claim, additionally including a test region (2) which includes a test for identifying triglycerides and additionally including a third channel (220), wherein said channel has a first end proximate the inlet (10) and a second end proximate said test region, and wherein the transfer element (200) allows capillary flow of blood plasma from the inlet along said channel to said test region.

10. A device as claimed in any preceding claim additionally including a region of said transfer element (200) between the inlet (10) and the end of each channel (210,220,230) proximate the inlet.

11. A device as claimed in any preceding claim, wherein the transfer element (200) has hydrophobic and hydrophilic regions which are patterned so as to define the at least one channel (210,220,230) and the at least one test region (1,2,3).

12. A device as claimed in claim 11, wherein the channel (210,220,230) and test region (1,2,3) are formed from the hydrophilic regions.

13. A device as claimed in any preceding claim, wherein the transfer element (200) is formed from a porous membrane.

14. A device as claimed in any preceding claim, additionally including a filter (100) for separating blood plasma from whole blood, the filter (100) being located on the opposite side of the inlet (10) to the at least one channel (210,220,230).

15. A device as claimed in any preceding claim, which has a single transfer element (200) which is substantially planar, and wherein the capillary flow of blood plasma is in the plane of the transfer element.

16. A method of testing blood plasma for cholesterol, including:
depositing a sample of blood plasma onto the device of any preceding claim, wherein the sample is flowed by capillary forces from the inlet (10) to the at least one test region (1,2,3) of said device through the at least one channel (210,220,230); and,
determining a concentration of cholesterol present in the sample according to a colourimetric analysis of the at least one test region.

## Patentansprüche

1. Vorrichtung zum Testen von Blutplasma auf Cholesterin, aufweisend einen Einlass (10)für Blutplasma,
mindestens einen Testbereich (3), der einen Test zur Identifizierung von Cholesterin hoher Dichte (HDL) umfasst,
erste und zweite gegenüberliegende Wände, die mindestens einen Kanal (230) definieren, wobei der Kanal ein erstes Ende in der Nähe des Einlasses und ein zweites Ende in der Nähe des Testbereichs aufweist,
ein Transferelement (200), das aus einem Material gebildet ist, das einen Kapillarfluss von Blutplasma vom Einlass entlang des Kanals zum Testbereich ermöglicht,
einen in dem Kanal befindlichen Reaktanten, der mit Lipoprotein niedriger Dichte (LDL)-Cholesterin in dem Blutplasma reagiert und verhindert, dass das LDL-Cholesterin den Testbereich (3) erreicht,
wobei der Kanal eine Vielzahl von Ecken (235) an einer oder beiden Wänden aufweist, wobei die Ecken ein Zickzackprofil definieren, das den Fluss des Blutplasmas beeinflusst, um die Reaktion des Plasmas mit dem Reaktanten zu fördern.

2. Vorrichtung nach Anspruch 1, wobei die Ecken (235) ein unregelmäßiges Zickzackprofil bilden.

3. Vorrichtung nach Anspruch 1 oder 2, wobei die Ecken (235) einen Innenwinkel von 70-90° und einen Außenwinkel von 90-140° bilden.

4. Vorrichtung nach einem der vorhergehenden Ansprüche, wobei die Breite des Kanals (230) über seine Länge nicht einheitlich ist.

5. Vorrichtung nach einem der vorhergehenden Ansprüche, wobei der Kanal (230) eine Dichte von Ecken (235) von mindestens zwei Ecken für jeden mm der Länge des Kanals aufweist.

6. Vorrichtung nach einem der vorhergehenden Ansprüche, wobei die Gesamtzahl der Ecken (235) mindestens 14, vorzugsweise 16 bis 32 und besonders bevorzugt 16 beträgt.

7. Vorrichtung nach einem der vorhergehenden Ansprüche, wobei die durchschnittliche Breite des Kanals (230) 18% bis 21% der Länge des Kanals beträgt, die maximale Breite des Kanals das 1.9 fache der durchschnittlichen Breite nicht übersteigt, die Mindestbreite des Kanals nicht weniger als 48 % der durchschnittlichen Breite beträgt, und die Tiefe des Kanals etwa 7 % der durchschnittlichen Breite beträgt.

8. Vorrichtung nach einem der vorhergehenden Ansprüche, die zusätzlich einen Testbereich (1), der einen Test zur Identifizierung von Gesamtcholesterin enthält, und zusätzlich einen zweiten Kanal (210) aufweist, wobei der Kanal ein erstes Ende in der Nähe des Einlasses (10) und ein zweites Ende in der Nähe des Testbereichs aufweist und wobei das Transferelement (200) einen Kapillarfluss von Blutplasma vom Einlass entlang des Kanals zum Testbereich ermöglicht.

9. Vorrichtung nach einem der vorhergehenden Ansprüche, die zusätzlich einen Testbereich (2) aufweist, der einen Test zur Identifizierung von Triglyceriden enthält, und die zusätzlich einen dritten Kanal (220) aufweist, wobei der Kanal ein erstes Ende in der Nähe des Einlasses (10) und ein zweites Ende in der Nähe des Testbereichs aufweist, und wobei das Transferelement (200) einen Kapillarfluss von Blutplasma vom Einlass entlang des Kanals zum Testbereich ermöglicht.

10. Vorrichtung nach einem der vorhergehenden Ansprüche, die zusätzlich einen Bereich des Transferelements (200) zwischen dem Einlass (10) und dem Ende jedes Kanals (210, 220, 230) in der Nähe des Einlasses umfasst.

11. Vorrichtung nach einem der vorhergehenden Ansprüche, wobei das Transferelement (200) hydrophobe und hydrophile Bereiche aufweist, die so strukturiert sind, dass sie den mindestens einen Kanal (210, 220, 230) und den mindestens einen Testbereich (1, 2, 3) definieren.

12. Vorrichtung nach Anspruch 11, wobei der Kanal (210, 220, 230) und der Testbereich (1, 2, 3) aus den hydrophilen Bereichen gebildet sind.

13. Vorrichtung nach einem der vorhergehenden Ansprüche, wobei das Transferelement (200) aus einer porösen Membran gebildet ist.

14. Vorrichtung nach einem der vorhergehenden Ansprüche, die zusätzlich einen Filter (100) zur Abtrennung von Blutplasma von Vollblut enthält, wobei der Filter (100) auf der dem mindestens einen Kanal (210, 220, 230) gegenüberliegenden Seite des Einlasses (10) angeordnet ist.

15. Vorrichtung nach einem der vorhergehenden Ansprüche, die ein einzelnes Transferelement (200) aufweist, das im Wesentlichen eben ist, und wobei der Kapillarfluss des Blutplasmas in der Ebene des Transferelements verläuft.

16. Verfahren zum Testen von Blutplasma auf Cholesterin, aufweisend:
Aufbringen einer Blutplasmaprobe auf die Vorrichtung nach einem der vorhergehenden Ansprüche, wobei die Probe durch Kapillarkräfte vom Einlass (10) zu dem mindestens einen Testbereich (1, 2, 3) der Vorrichtung durch den mindestens einen Kanal (210, 220, 230) fließt; und,
Bestimmen der in der Probe vorhandenen Cholesterinkonzentration anhand einer kolorimetrischen Analyse der mindestens einen Testregion.

## Revendications

1. - Dispositif de test de plasma sanguin pour cholestérol, comprenant
une entrée (10) pour plasma sanguin,
au moins une région de test (3) qui comprend un test pour identifier du cholestérol à lipoprotéines de haute densité (LHD),
des première et seconde parois opposées définissant au moins un canal (230), ledit canal ayant une première extrémité à proximité de l'entrée et une seconde extrémité à proximité de la région de test,
un élément de transfert (200) qui est formé à partir d'un matériau qui permet un écoulement capillaire de plasma sanguin depuis l'entrée le long dudit canal jusqu'à la région de test,
un réactif situé dans ledit canal qui réagit avec du cholestérol à lipoprotéines de basse densité (LED) dans ledit plasma sanguin, et empêche le cholestérol LED d'atteindre la région de test (3),
dans lequel ledit canal possède une multiplicité de coins (235) sur l'une ou les deux desdites parois, lesdits coins définissant un profil en zigzag qui affecte l'écoulement de plasma sanguin de manière à favoriser la réaction du plasma avec ledit réactif.

2. - Dispositif selon la revendication 1, dans lequel les coins (235) définissent un profil en zigzag irrégulier.

3. - Dispositif selon la revendication 1 ou 2, dans lequel les coins (235) définissent un angle intérieur de 70 à 90° et un angle extérieur de 90 à 140°.

4. - Dispositif selon l'une quelconque des revendications précédentes, dans lequel la largeur du canal (230) est non-uniforme le long de sa longueur.

5. - Dispositif selon l'une quelconque des revendications précédentes, dans lequel le canal (230) comprend une densité de coins (235) d'au moins deux coins pour chaque mm de la longueur du canal.

6. - Dispositif selon l'une quelconque des revendications précédentes, dans lequel le nombre total de coins (235) est d'au moins 14, de préférence de 16 à 32, et plus préférentiellement de 16.

7. - Dispositif selon l'une quelconque des revendications précédentes, dans lequel la largeur moyenne du canal (230) va 18% à 21% de la longueur du canal, la largeur maximale du canal n'excède pas 1,9 fois la largeur moyenne, la largeur minimale du canal n'est pas inférieure à 48% de la largeur moyenne, et la profondeur du canal est d'environ 7% de la largeur moyenne.

8. - Dispositif selon l'une quelconque des revendications précédentes, comprenant en outre une région de test (1) qui comprend un test pour identifier du cholestérol total, et comprenant en outre un deuxième canal (210), dans lequel ledit canal possède une première extrémité à proximité de l'entrée (10) et une seconde extrémité à proximité de ladite région de test, et dans lequel l'élément de transfert (200) permet un écoulement capillaire de plasma sanguin depuis l'entrée le long dudit canal jusqu'à ladite région de test.

9. - Dispositif selon l'une quelconque des revendications précédentes, comprenant en outre une région de test (2) qui comprend un test pour identifier des triglycérides, et comprenant en outre un troisième canal (220), dans lequel ledit canal possède une première extrémité à proximité de l'entrée (10) et une seconde extrémité à proximité de ladite région de test, et dans lequel l'élément de transfert (200) permet un écoulement capillaire de plasma sanguin depuis l'entrée le long dudit canal jusqu'à ladite région de test.

10. - Dispositif selon l'une quelconque des revendications précédentes, comprenant en outre une région dudit élément de transfert (200) entre l'entrée (10) et l'extrémité de chaque canal (210, 220, 230) à proximité de l'entrée.

11. - Dispositif selon l'une quelconque des revendications précédentes, dans lequel l'élément de transfert (200) possède des régions hydrophobes et hydrophiles qui sont modélisées de façon à définir l'au moins un canal (210, 220, 230) et l'au moins une région de test (1, 2, 3).

12. - Dispositif selon la revendication 11, dans lequel le canal (210, 220, 230) et la région de test (1, 2, 3) sont formés à partir des régions hydrophiles.

13. - Dispositif selon l'une quelconque des revendications précédentes, dans lequel l'élément de transfert (200) est formé à partir d'une membrane poreuse.

14. - Dispositif selon l'une quelconque des revendications précédentes, comprenant en outre un filtre (100) pour séparer le plasma sanguin du sang total, le filtre (100) étant situé sur le côté de l'entrée (10) opposé à l'au moins un canal (210, 220, 230).

15. - Dispositif selon l'une quelconque des revendications précédentes, comprenant un seul élément de transfert (200) qui est sensiblement plan, et dans lequel le flux capillaire de plasma sanguin s'effectue dans le plan de l'élément de transfert.

16. - Procédé de test de plasma sanguin pour cholestérol, comprenant :
déposer un échantillon de plasma sanguin sur le dispositif selon l'une quelconque des revendications précédentes, l'échantillon s'écoulant par des forces capillaires depuis l'entrée (10) jusqu'à l'au moins une région de test (1, 2, 3) dudit dispositif à travers l'au moins un canal (210, 220, 230) ; et,
déterminer une concentration de cholestérol présent dans l'échantillon en fonction d'une analyse colorimétrique de l'au moins une région de test.
